Europäisches Patentamt

European Patent Office

Office européen des brevets

(11) Publication number: **0 338 497**
**A2**

(12) **EUROPEAN PATENT APPLICATION**

(21) Application number: 89106899.1

(22) Date of filing: 18.04.89

(51) Int. Cl.⁴ **C12N 5/00 , C12P 21/00 , C12N 15/00 , G01N 33/577**

The applicant has filed a statement in accordance with Rule 28 (4) EPC (issue of a sample only to an expert). Accession number(s) of the deposit(s):BP-1587,BP-1589,BP-1591,BP-1813,-BP-1814,BP-1840

(30) Priority: 22.04.88 JP 98278/88
17.06.88 JP 148160/88

(43) Date of publication of application:
25.10.89 Bulletin 89/43

(84) Designated Contracting States:
AT BE CH DE ES FR GB GR IT LI LU NL SE

(71) Applicant: TAKEDA CHEMICAL INDUSTRIES, LTD.
3-6, Doshomachi 2-chome Chuo-ku
Osaka 541(JP)

(72) Inventor: Iwasa, Susumu
21-2, Ohsumigaoka 1-chome Tanabe-cho
Tsuzuki-gun Kyoto 610-03(JP)
Inventor: Toyoda, Yukio
8-8, Mizudocho 1-chome
Amagasaki Hyogo 661(JP)
Inventor: Nakata, Mitsugu
22-5, Kiyomidai 1-chome
Kawachinagano Osaka 586(JP)

(74) Representative: von Kreisler, Alek et al
Patentanwälte Von
Kreisler-Selting-Werner-Schönwald-Fues-Da-llmeyer Deichmannhaus
D-5000 Köln 1(DE)

(54) Bispecific hybrid monoclonal antibody.

(57) Disclosed are a bispecific hybrid monoclonal antibody and a polydoma, preferably a trioma, for producing it. One molecule of the bispecific hybrid monoclonal antibody is capable of linking to both human lymphotoxin and an enzyme such as horseradish peroxidase, a fluorescent substance or a radionuclide.

The bispecific hybrid monoclonal antibody is produced from the polydoma, particularly the trioma obtained by fusing an anti-human lymphotoxin-producing hybridoma with spleen cells of mouse immunized with horseradish peroxidase.

Using this bispecific hybrid monoclonal antibody, an immunodiagnostic method for detecting the human lymphotoxin specifically, rapidly and easily, is provided.

EP 0 338 497 A2

## BISPECIFIC HYBRID MONOCLONAL ANTIBODY

Background of the Invention

The present invention relates to a bispecific hybrid monoclonal antibody and a polydoma (hybrid hybridoma) for production thereof; more particularly to a polydoma for producing a hybrid monoclonal antibody specific for both human lymphotoxin (hereinafter occasionally referred to as hLT) and a substance which can permit detection of or analysis for hLT; as well as the monoclonal antibody (hereinafter occasionally referred to as MoAb) produced therefrom.

This invention is further directed to immunodiagnostic methods for determining hLT specifically, rapidly and easily by use of hybrid monoclonal antibodies having the above characteristics.

Human lymphotoxin is a lymphokine consisting of 171 amino acid residues, which is believed to act as an antitumor agent in a manner similar to that of tumor necrosis factor (hereinafter occasionally referred to as TNF), due to its selective antitumor activity. Since Grey et al. succeeded in the expression of hLT in Escherichia coli using gene manipulation techniques [Nature, 312, 721, 724 (1984)], its mass production has become possible and the ways have been opened for its clinical applications. Therefore, it has become important to establish a method for detecting and measuring hLT.

At present, the bioassay using L929 cells is most widely used as a method for detecting hLT [B. R. Spofford et al., J. Immunol., 112, 211 (1974)]. This method is however complicated and time-consuming. Further, in this method, hLT can not be distinguished from similar proteins such as TNF or other cytotoxic substances. There has been therefore awaited a method for determining hLT more rapidly, easily and specifically.

Bringman et al. reported that an antibody against hLT was prepared and an enzyme-linked immunosorbent assay (hereinafter occasionally referred to as ELISA) using the antibody was developed, by which hLT could be more easily determined than by the bioassay [Hybridoma, 6, 489 (1987)]. They determined hLT by a sandwich method, using both a rabbit polyclonal antibody (hereinafter occasionally referred to as PoAb) and a mouse MoAb against hLT. However, this method requires 6 to 8 hours to perform and complicated procedures for labeling either of the antibodies with a marker such as an enzyme, a fluorescent substance or a radionuclide. These labeling procedures require a chemical binding method and are accompanied by a reduction of antigen-binding activity of the antibodies, aggregation of the macromobecules, and nonspecific adsorption to the solid phase used in the assay, and other problems. The labeling procedures also decrease the stability of the labeled antibody.

Further, in the sandwich method, hLT in a test solution is first linked to an anti-hLT antibody in a solid phase. After washing, a labeled antibody linked to a marker is added thereto, followed by washing again. Then, the determination of the marker is carried out. At least two steps of linking reactions and washing procedures are thus required. This method therefore requires so much manipulation that it cannot be said to be an easy method.

On the other hand, recent advances in preparation techniques for hybridomas enabled new types of polydomas such as triomas and tetraomas to appear [C. Milstein et al., Nature, 305, 537 (1983), M. R. Suresh et al., Proc. Natl. Acad. Sci. U.S.A., 83, 7989 (1986)] and bispecific antibodies having new functions to be prepared [Japanese Patent Unexamined Publication No. 63-12276 (Hybritech), U. S. Patent No. 4,714,681 (Univ. of Texas System Cancer Center)]. In this specification, amino acids and peptides are indicated by the abbreviations adopted by IUPAC-IUB Committee of Biochemistry Nomenclature (CBN). For example, the following abbreviations are used:

Gln: Glutamine residue
Asp: Aspartic acid residue
Pro: Proline residue
Tyr: Tyrosine residue
Val: Valine residue
Lys: Lysine residue
Glu: Glutamic acid residue
Ala: Alanine residue
Asn: Asparagine residue
Leu: Leucine residue
Phe: Phenylalanine residue
Gly: Glycine residue

His: Histidine residue
Ser: Serine residue
Thr: Threonine residue
Ile: Isoleucine residue
Trp: Tryptophan residue
Arg: Arginine residue
Met: Methionine residue

When the optical isomer is capable of existing with respect to the amino acids and the like, the L-form is represented unless otherwise specified.

Also in this specification, polymers or oligomers of deoxyribonucleic acid (hereinafter referred to as DNA) are indicated by the sequence of the following abbreviations:

A: 2′-Deoxyadenylic acid residue
C: 2′-Deoxycytidylic acid residue
G: 2′-Deoxyguanylic acid residue
T: Thymidylic acid residue

Unless otherwise stated, the direction from the left to the right in sequence indicates the direction from 5′-position to the 3′-position.

Brief Description of the Drawings

Fig. 1 is a graph showing dilution curves of hybrid antibody PLT-109 obtained by the ELISAs described in Reference Examples 5 (●), 6 (o) and 7 (△);

Fig. 2 is a graph showing a standard curve of hLT obtained by the ELISA using hybrid antibody PLT-109 described in Example 2;

Fig. 3 is a graph showing a correlation between the bioassay described in Reference Example 1 and the ELISA described in Example 2;

Figs. 4 and 5 are graphs showing standard curves of hLT obtained by the ELISAs using hybrid antibody PLT-109 described in Examples 3 and 4, respectively;

Figs. 6(A), 6(B) and 6(C) are graphs showing high performance liquid chromatograms of a hydroxyapatite column obtained in Example 1;

Fig. 7 is a diagram showing the results of SDS-PAGE obtained with respect to the peaks 1 to 7 shown in Fig. 6(A) and anti-hLT MoAb LT3-11;

Fig. 8 is a graph showing a correlation between the bioassay described in Reference Example 1 and the ELISA described in Example 5; and

Fig. 9 is a graph showing a comparison of the hLT-neutralizing activity of hybrid antibody PLT-109 (▲) with that of parent antibody LT3-11 (o).

Summary of the Invention

It is therefore a primary object of the present invention to provide a hybrid MoAb which eliminates the need for the procedure for chemically linking a marker to an antibody or an antigen, the above procedure being indispensable in known immunoassays. The present invention makes it possible to simplify the assay manipulation and to shorten the manipulating time.

It is another object of the present invention to provide immunodiagnostic methods for detecting and measuring hLT by use of the above hybrid MoAb.

Other objects of this invention will be apparent from the following description and the accompanying drawings.

The present invention provides a bispecific hybrid MoAb specific both for hLT and for a substance which can measure or permit detection of hLT, such as an enzyme, a fluorescent substance or a radionuclide. The present invention also provides polydomas for producing such bispecific hybrid MoAbs; as well as methods for making and using such polydomas and hybrid MoAbs.

More particularly, there is provided a bispecific hybrid MoAb which is produced by a trioma obtained by the fusion of an anti-hLT antibody-producing hybridoma with spleen cells from a mouse immunized with horseradish peroxidase (hereinafter occasionally referred to as HRP).

The present invention further includes immunodiagnostic methods for determining hLT specifically, rapidly and easily by use of the above hybrid MoAb.

3

## Detailed Description of the Invention

Preferably, hybridomas producing anti-hLT neutralizing antibodies are used for preparation of the bispecific hybrid MoAb-producing polydomas of the present invention.

In order to obtain such hybridomas, N-terminal portion-deleted LT may be used as an immunogen. The immunogen can be used in mice, for example, to develop the anti-hLT antibody-producing cells. The scope of the present invention is not intended to be limited to anti-hLT antibody-producing hybridomas derived from immunized mice, however, since such hybridomas may also be derived from a variety of immunized mammalian species, including rat, rabbit, human, and others. The immunogen can be prepared, by expression in, for example, bactrial cells (e.g. Escherichia coli) in the following manner:

1. m-RNAs can be collected by known methods from human peripheral blood lymphocytes in which the synthesis of LT is induced by 12-o-tetradecanoylphorbol-13-acetate (TPA) and concanavalin A (ConA). A cDNA library of approximately 5 X 10^5 recombinants can be prepared therefrom by known methods.

2. An oligonucleotide ranging in size from 10-mer to 50-mer coding for a partial peptide chain of LT is synthesized. By using this oligonucleotide as a probe, screening of LT cDNA can be conducted. For example. when a synthetic oligo nucleotide of the 18-mer (TCCAAAGAAGACAGTACT) at coding for a portion of the C-terminal of hLT is used, about 50 hLT cDNA clones can be obtained.

3. The nucleotide sequences of plasmids which are isolated from the LT cDNA clones are determined. A plasmid coding for the amino acid sequence of LT is selected, cleaved with an appropriate restriction enzyme, and then inserted into a suitable expression vector by known techniques. Thus, a recombinant DNA containing the DNA fragment can be prepared.

4. Bacterial hosts, for example, Escherichia coli, are transformed by using the vector prepared in item 3. Consequently, bacterial strains containing the DNA coding for LT can be obtained.

5. After the transformants prepared in 4 are cultivated and the plasmids are isolated, DNAs coding for N-terminal-deleted LT can be prepared as follows:

(A) A restriction enzyme NsiI recognition site is located on the human LT gene in the region coding for methionine-histidine situated in the 20th-21st positions from the N-terminus. A DNA fragment coding for hLT(20-171) can be obtained by digesting the LT gene with NsiI at that site. A DNA fragment coding for LT (20-171) is produced by linking an appropriate adapter containing the following sequence to the above fragment.

$$
\begin{array}{ll}
5' & 3' \\
\text{G A A T T C A T G C C} \\
\text{C T T A A G T} \\
3' \qquad\qquad 5'
\end{array}
$$

The resulting DNA fragment is inserted into an appropriate vector:

(B) Alternatively a chemically synthesized DNA fragment can be linked with the DNA fragment coding for N-terminal portion-deleted LT which has been cleaved with NsiI. In this case, a DNA fragment coding for a peptide chain corresponding to hLT (10-20) or a DNA fragment coding for a peptide chain in which one or more amino acids or peptide of a part of the hLT (10-20) peptide chain is eliminated or substituted with another amino acid or peptide can be used. These may be synthesized so as to maintain the reading frame correctly.

(C) Similarly, a restriction enzyme PvuII recognition site is located on the human LT gene in the region coding for serine-alanine situated in the 9th-10th positions from the N-terminus. A DNA fragment coding for N-terminal portion-deleted LT can also be obtained by digesting the LT gene with PvuII. A DNA fragment coding for LT (10-171) is produced by linking an appropriate adapter containing the following sequence with the above fragment.

$$
\begin{array}{ll}
5' & 3' \\
\text{G A A T T C A T G C} \\
\text{C T T A A G T A C G} \\
3' \qquad\qquad 5'
\end{array}
$$

The produced DNA fragment is inserted into an appropriate vector.

(D) A DNA fragment coding for N-terminal-deleted LT which has been cleaved with PvuII can be further treated with an exonuclease such as nuclease BAL31 to remove the region coding for 1-10 amino acids from the PvuII fragment. Then, a DNA fragment coding for LT (X-171), X = 10 to 20, can be prepared by selecting the DNA fragment in which the reading frame of the gene is correctly maintained.

(E) The technique of site-directed mutagenesis [M. Smith and S. Gillam, Genetic Engineering, 3, 1 (1981); T. Maniatis et al., Molecular Cloning, Cold Spring Harbor Laboratory, P. 312 (1982)] may also be applied. Namely, after cleavage with PvuII, the DNA fragment coding for N-terminal portion-deleted LT is inserted into vector M13, with which Escherichia coli JM103 (Pharmacia P-L Biochemicals) is infected. After cultivation, M13 phages released in the broth are precipitated with polyethylene glycol and treated with phenol, whereby a single stranded M13 phage DNA can be obtained.

Then, the DNA coding for a peptide chain in which an amino acid or peptide of a part of the peptide chain from alanine situated in the 10th position to methionine situated in the 20th position from the N-terminus of LT is eliminated or substituted with another amino acid or peptide is prepared by a chemical synthesis method and used as a primer. This primer is mixed with the M13 phage DNA fragment previously prepared to form a double-stranded DNA by DNA polymerase I large fragment. Thereafter, the cyclization thereof can be achieved by T4 DNA ligase. This circular DNA is introduced into Escherichia coli JM103, and the released M13 phage DNAs are transferred onto a filter. Then, plaque hybridization [T. Maniatis et al., Molecular Cloning, Cold Spring Harbor Laboratory, p.312 (1982)] is carried out by using the synthesized primer labeled with $^{32}$P. A DNA is prepared from the phage from which a strong signal is detected, and cut out with an appropriate restriction enzyme. The DNA fragment thus obtained is inserted into a plasmid to provide a DNA coding for modified N-terminal portion-deleted LT.

Plasmid DNA, cosmid DNA, phage DNA containing the whole or a part of the human LT gene can be used as a template DNA for the site-directed mutagenesis. M13 phage or ØX174 phage is desirable as a template DNA, because a single-stranded DNA can be easily prepared therefrom. For example, when M13 phage or ØX174 phage containing the whole or a part of the human LT gene is used, phage particles present in the broth are precipitated with polyethylene glycol, deproteinized by phenol treatment, and then precipitated with ethanol to obtain single-stranded DNA. When a double-stranded plasmid DNA or cosmid DNA containing the whole or a part of the human LT gene is used as a template DNA, the double-stranded DNA is subjected to heat treatment at 100°C for 1 to 10 minutes, preferably 3 to 5 minutes, and then cooled rapidly with ice water to be denatured to single-stranded DNA, prior to use.

The primer for the site-directed mutagenesis may have any DNA sequence so long as it has a DNA sequence to be changed and functions as the primer on the DNA synthesis through hybridization with a template DNA. The primer may be produced by any method, but it is desirable to prepare a single-stranded DNA having an appropriate sequence by a chemical synthesis method. Such a primer is mixed with the single-stranded DNA previously prepared, and repaired to a double-stranded DNA by DNA polymerase I large fragment. Thereafter, the cyclization can be achieved by T4 DNA ligase. After the circular DNA thus obtained is introduced into Escherichia coli, plaque hybridization [T. Maniatis et al., Molecular Cloning, Cold Spring Harbor Laboratory, p.312 (1982)] or colony hybridization (ibid. p. 326) is carried out, using the primer labeled with a radioisotope as a probe, whereby the desired mutant can be selected. By using phage DNA or plasmid DNA prepared from the plaque or the colony thus obtained, an N-terminal portion-deleted LT gene can be prepared.

Then, a recombinant DNA capable of expressing the DNA coding for N-terminal portion-deleted LT can be constructed, by inserting the obtained DNA fragment coding for N-terminal portion-deleted LT at the 3'-terminus of the promoter region which functions in the host to be used. Hosts include Escherichia coli, Bacillus subtilis, yeast or animal cells. Any promotor region is available, if the region contains a site necessary for initiating the mRNA synthesis by linkage of RNA polymerase. The promoter used is not limited to the trp promoter (trp-p). For example, there may be used the recA promoter (Japanese Patent Unexamined Publication No. 59- 65099/1984), the lac promoter, the λP$_L$ promoter or the like.

For example, when Escherichia coli is used as a host, the recombinant DNA capable of expressing the DNA sequence coding for N-terminal portion-deleted LT can be constructed by inserting the DNA fragment coding for N-terminal portion-deleted LT at the 3'-terminus of a vector containing a promoter region capable of functioning in Escherichia coli. When Escherichia coli plasmids such as pBR322, pBR325, ptrp781, pUC8, pUC9, pJB8 can be used as vectors, and the DNA fragment coding for N-terminal portion-deleted LT is inserted into the selected vector using T4 DNA ligase. Escherichia coli strains such as C600, MM294, DH1, W3110, PR1 or PR13 can be transformed by the vector containing the N-terminal portion-deleted hLT according to the method of S. N. Cohen et al., Proc. Natl. Acad. Sci. U.S.A., 69, 2110 (1972) or a similar method thereto.

The transformant carrying the recombinant plasmid DNA containing the DNA fragment coding for N-terminal-deleted LT obtained can be selected by phenotype, for example, an ampicillin resistance type, a tetracycline resistance type or a resistance type to both of these drugs.

The selected transformant is cultivated in a known medium, for example, L-broth, Penassay broth and M-9 medium containing glucose and Casamino Acids [J. Miller, Experiments in Molecular Genetics, p. 431-433 (Cold Spring Harbor Laboratory, New York, 1972)]. In order to allow the promoter to act efficiently, a drug such as $3\beta$-indolylacrylic acid may be added thereto if necessary.

Cultivation of the above transformant is generally carried out at 15 to 43°C, preferably 28 to 40°C, for 2 to 24 hours, preferably 4 to 16 hours, with aeration or agitation if necessary.

After cultivating, the cells are collected by a known method. In case of the transformant of Escherichia coli, the cells are suspended in an appropriate buffer, for example, Tris-hydrochloric acid buffer (pH 7.5), and disrupted by ultrasonic treatment, lysozyme and/or freeze-thawing. Thereafter, the supernatant containing N-terminal portion-deleted LT is obtained by centrifugation. There can be preferably employed a method in which the collected cells are suspended in a buffer solution, to which lysozyme is added, and incubated at 0 to 10°C for 10 minutes to 3 hours, and the resulting culture is treated with ultrasonication at 0 to 10°C for 30 seconds to 5 minutes and then centrifuged to obtain the supernatant.

Separation and purification of N-terminal portion-deleted LT from the extract can be carried out, for example, by gel filtration, hydroxyapatite column chromatography, ion- exchange column chromatography, ultracentrifugation or affinity chromatography using anti-hLT antibody.

The N-terminal portion-deleted LT described above is inoculated in animals to induce anti-hLT antibodies. The animals for inoculation include, for example, rabbit, rat, mouse and guinea pig. When MoAb is prepared, mouse is preferably used.

The inoculation is performed according to a standard method. For example, 1 to 100 μg each time, preferably 10 to 25μg each time of the N-terminal portion-deleted LT emulsified with an equal volume (0.1 ml) of physiological saline and Freund's complete adjuvant is inoculated into a mouse subcutaneously in the back or the abdomen or intraperitoneally, 3 to 6 times every 2 to 3 weeks.

Individuals having a high antibody titer are selected, and their spleens or lymphatic nodes are collected therefrom 3 to 5 days after the final immunization. Then, antibody-producing cells contained therein are fused with myeloma cells. The fusing operation may be conducted according to a known method. Fusogens include polyethylene glycol (hereinafter referred to as PEG) and Sendai virus; PEG having a molecular weight of 1,000 to 6,000 is preferably used. The myeloma cells include NS-1, P3UI and Sp2/0. Particularly, P3UI is preferably used. The preferable ratio of the number of the spleen cells to that of the myeloma cells is 1:1 to 10:1. PEG is added to the cells in a concentration of 10 to 80%, and the resulting mixture is incubated at 20 to 37°C, preferably 30 to 37°C, for 3 to 10 minutes.

Hybridoma cells having positive antibody activity, which is selected an HAT (hypoxanthine, aminopterin, and thymidine) medium are subjected to screening which can occur by various methods. For example, an ELISA can be performed by adding the hybridoma culture supernatant to a microtiter plate on which human LT is adsorbed, then adding the anti-mouse immunoglobulin antibody labeled with horseradish peroxidase thereto, and detecting the anti-human LT monoclonal antibody bound to the plate as the solid phase. The antibody titers of the hybridoma culture supernatants are determined by the limiting dilution method, and hybridoma cells which stably produce antibody having a high titer are selected and cloned. Thus, the desired monoclonal anti-hLT antibody-producing hybridoma cells can be obtained.

The anti-hLT antibody-producing hybridoma cells of the present invention include mouse hybridoma LT3-11 and LT3-135.

In order to prepare the polydoma cells which produce the bispecific hybrid MoAbs of the present invention, the above HAT-resistant anti-hLT antibody-producing hybridoma cells are conditioned stepwise in a culture solution containing 8-azaguanine (hereinafter occasionally referred to as AZG) to turn them HAT-sensitive, and then fused with antibody-producing cells, for example spleen cells, of mouse immunized with HRP.

The methods for preparing the hybrid antibody-producing polydoma cells can conceivably include fusion of antibody-producing cells derived from mouse immunized with hLT and anti-HRP antibody-producing hybridoma cells (preparation of trioma cells), the fusion between anti-hLT antibody-producing hybridoma cells and anti-HRP antibody-producing hybridoma cells (preparation of tetraoma cells) and the like, in addition to the method described above.

For cell fusions in this manipulation, there are used fusogens such as Sendai virus, PEG, and electrical stimulation. Preferably, PEG is used. One example thereof will hereinafter be described. However, the scope of the present invention is not limited thereto. Namely, PEG having a molecular weight of about 1,000 to 6,000 is used in a concentration of about 10 to 80%. The reaction time is about 0.5 to 30 minutes. For example, PEG 6,000 is allowed to contact with cells in a concentration of about 35 to 55% at 37°C for about 4 to 10 minutes to perform the fusion efficiently.

Selection of the trioma cells to be obtained can be carried out in HAT medium. Hybrid hybridoma cells are selected and cloned which secrete a hybrid antibody capable of linking to both hLT and HRP. After selection with HAT medium the polydoma cells are conditioned with drugs such as 8-AZG, 6-thioguanine (6-TG) and 5-bromodeoxyuridine to obtain the respective drug-resistant strains. Further, various selection media are used for the introduction of a new marker into fused cells. Examples of such media include neomycin-added media and hygromycin B-added media [B. Sugden et al., Mol. Cell. Biol., 5, 410 (1985)].

The hybrid antibody-producing polydoma cells can be screened by various methods. For example, the following methods can be suitably used in combination:

(1) An ELISA for screening the above anti-hLT antibody-producing hybridoma cells;

(2) An ELISA which comprises adding a polydoma culture supernatant to a microplate of which solid phase is allowed to adsorb an anti-mouse immunoglobulin antibody and then adding HRP thereto, followed by detection of an anti-HRP antibody linked to the plate solid phase;

(3) An ELISA which comprises adding a test culture supernatant to a microplate of which solid phase is allowed to adsorb hLT and then adding HRP thereto, followed by detection of a bispecific hybrid antibody.

Polydoma cells having positive antibody activity are immediately subjected to cloning, which is usually conducted with ease by a limiting dilution method. The antibody titer of the cloned polydoma culture supernatant is determined by the above-mentioned method, and polydoma cells which stably produce an antibody having a high titer are selected. Thus, the desired monoclonal hybrid antibody-producing polydoma cells can be obtained. The hybrid antibody-producing polydomas prepared by the methods described above include the trioma PLT-109 of the present invention, the detailed preparation of which is shown in Example 1.

The above-mentioned polydoma cells of the present invention can be cultivated in liquid media or in peritoneal cavities of animals (for example, in peritoneal cavities of mammals such as mouse) by methods known in the art. The antibodies in culture solutions or ascites fluid can be purified by using biochemical techniques known in the art in combination. The hybrid MoAb obtained as described above can be treated with a protease (for example, pepsin) to produce $F(ab')_2$ fragments having dual binding capacity for hLT and HRP. These $F(ab')_2$ fragments may be used for the same purposes as those of the intact MoAb of the present invention.

For example, a cell culture solution or ascites is centrifuged to obtain a supernatant. The supernatant is taken out and salted out, usually by use of ammonium sulfate or sodium sulfate. The resulting protein precipitate is dissolved in an appropriate solution. After dialysis, the solution is submitted to column chromatography such as ion-exchange column, gel filtration column, Protein A column or hydroxyapatite column chromatography. Thus, the desired antibody can be separated and purified. By such separation and purification procedures, for example, about 1 to 5 mg of the hybrid MoAb having purity of more than 70% by a protein weight ratio can be obtained from 1 l(liter) of the culture supernatant. Further, 3 to 10 mg of the similar antibody can be obtained from 20 ml of ascites fluid.

In the immunodiagnostic methods of hLT of the present invention, various assay systems are used. For example, there are (1) a method for determining hLT in a test solution by adding fixed amounts of the hybrid antibody of the present invention, hLT linked to a solid phase and an enzyme to a test solution containing an unknown amount of hLT, and measuring the activity of the enzyme linked to the solid phase, and (2) a method for determining hLT in a test solution by adding a hLT-containing test solution, fixed amounts of the hybrid antibody of the present invention and an enzyme to a solid phase to which an anti-hLT antibody is linked, wherein the antigen-recognition site of the above anti-hLT antibody and that of the hybrid antibody of the present invention do not overlap each other. In each method, the determination can be easily carried out in a short time, compared with previously known determination methods. For example, according to the method (1), the concentration of hLT can be determined within 1 to 2 hours. It is not necessary to allow the enzyme to link chemically to the antibody or the antigen in advance. Since, the free enzyme can be used. Moreover, a purified enzyme preparation is not necessarily required to be used in this case.

As test samples used in the assays of the present invention, any of serum, plasma, urine and tissue extract can be measured.

Any solid phase anti-hLT antibody may be used in the assay system of the method (2), so long as the antigen-recognition site thereof and that of the hybrid antibody of the present invention do not overlap each other. Preferably rabbit PoAbs prepared by using the C-terminal peptide of hLT as an immunogen are used. Examples of such anti-hLT C-terminal peptide antibodies include LT-R1, LT-R2 and LT-R3. For preparation of anti-hLT PoAbs, the following peptide is preferable as the C-terminal peptide used for the immunogen:

H-R-Ser-Thr-Val-Phe-Phe-Gly-Ala-Phe-Ala-Leu-OH wherein R is a peptide chain represented by Leu-Thr-Gln-Gly-Asp-Gln-Leu-Ser-Thr-His-Thr-Asp-Gly-Ile-Pro- His-Leu-Val-Leu-Ser-Pro or a portion thereof.

The above C-terminal peptide is prepared by known conventional peptide synthesis methods [Schroder and Lubke, The Peptides, vol. 1, Academic Press, New York, U.S.A. (1986) or Izumiya et al., Peptide Synthesis, Maruzen (1975)].

As the substances which can detect hLT in the present invention, there are used enzymes, fluorescent substances, radionuclides or the like. In particular, enzymes are frequently selected for their sensitivity, safety and ease of handling. The enzymes include HRP, $\beta$-galactosidase and alkaline phosphatase. Preferably, HRP is frequently used.

In the assays described above, the bispecific hybrid antibodies of the present invention can detect and measure hLT specifically, rapidly and easily, and hence are very useful for the clinical measurement of hLT having properties superior to those of known MoAbs.

The present invention will hereinafter be described in detail with the following Reference Examples and Examples. It is understood of course that these Reference Examples and Examples are not intended to limit the scope of the invention.

In carrying out the present invention, preparation of recombinant DNA and insertion of the recombinant into the microorganism were conducted according to the following experimental textbooks unless otherwise stated:

(1) T. Maniatis, E. F. Fritsch and J. Sambrook, Molecular Cloning, published by Cold Spring Harbor Laboratory (U.S.A.)

(2) Edited by Y. Takagi, Gene Manipulation Experimental Method, published by Kodansha (Japan)

The transformants and the animal cells used in the present invention are deposited in the deposition institutes as shown in the following table:

| Microorganism, animal cell | IFO (IFO No.) | FRI (FERM No.) |
|---|---|---|
| Escherichia coli DH1/pTB618 | 14542 | BP-1587 |
| Escherichia coli C600/pTB622 | 14544 | BP-1589 |
| Escherichia coli DH1/ptrp781 | 14546 | BP-1591 |
| Mouse-mouse hybridoma LT3-11 | 50167 | BP-1813 |
| Mouse-mouse hybridoma LT3-135 | 50164 | BP-1814 |
| Hybrid-hybridoma PLT1-109 | 50166 | BP-1840 |
| IFO: The Institute for Fermentation, Osaka, Japan | | |
| FRI: Fermentation Research Institute, Agency of Industrial Science and Technology, Ministry of International Trade and Industry, Japan | | |

Reference Example 1

Evaluation of Cytotoxic Activity against L929 Cells

The cytotoxicity of LT was measured by a method similar to that described in J. Immunol., 126, 235 (1981) or J. Immunol. Methods, 70, 257 (1984), using L929 cells. Namely, 50 $\mu$l of L929 cells suspended in a concentration of $4 \times 10^5$ cells/ml in RPMI 1640 medium supplemented with 10% fetal calf serum (FCS) containing 4 $\mu$g/ml of mitomycin C were added to 50 $\mu$l of a sample serially two-fold diluted with RPMI 1640 medium supplemented with 10% FCS by using a microplate for tissue culture (Flow Laboratory) having 96 wells, and then cultivated in 5% carbon dioxide gas at 37° C for 48 hours. After completion of the cultivation, the live cells were dyed with dimethylthiazolyl diphenyltetrazolium bromide (MTT), and dissolved with 10% SDS-0.01N HCl. Thereafter, the absorbance at 590 nm was measured with Titertek Multiscan (Flow Laboratory). The absorbance obtained is proportional to the number of live cells. The amount of biological activity required for killing 50% of L929 cells was defined as 1 unit/ml, and the biological activity of the sample was represented by unit/ml.

Reference Example 2

Collection of Natural LT

In accordance with the method described in Hinuma et al., Microbiol Immunol., 28, 935 (1984), normal human peripheral blood lymphocytes activated by TPA and ConA were cultivated in RPMI 1640 medium containing 10% FCS in 5% carbon dioxide gas at 37°C for 2 to 3 days, and a solution containing LT was obtained from the culture supernatant.

Reference Example 3

Preparation of Escherichia coli Strains for Transformation

Each colony of Escherichia coli strains DH1, C600 and MM294 was cultivated in 10 ml of SOB medium [experimental textbook (1), p. 69], until the absorbance at 550 nm reached 0.3. After being cooled with ice, the culture was centrifuged. Then, the resulting cells were washed with 5 ml of 10 mM NaCl. The cells were resuspended in 5 ml of 50 mM $CaCl_2$ and allowed to stand for 15 minutes under ice cooling. After centrifugation, the cells were suspended in 0.5 ml of 50 mM $CaCl_2$ and used immediately thereafter.

Reference Example 4

Preparation of N-Terminal Deleted hLT

(1) Preparation of cDNA Library by Using mRNA Derived from Human Lymphocytes.

The lymphocytes prepared from human peripheral blood were cultivated in RPMI 1640 medium (supplemented with 10% FCS) containing TPA (15 ng/ml) and ConA (40 µg/ml) at 37°C to induce LT. After 24 hours, $1 \times 10^{10}$ cells of the induced human lymphocytes were denatured by disruption with a Teflon homogenizer in a solution containing 5 M guanidine thiocyanate, 5% mercaptoethanol, 50 mM Tris-HCl (pH 7.6) and 10 mM EDTA. Thereafter, sodium N-lauroylsarcosinate was added thereto to a concentration of 4%, and the homogenized mixture was layered on 6 ml of 5.7 M cesium chloride solution (5.7 M cesium chloride and 0.1 M EDTA), and then centrifuged by a Beckmann SW28 rotor at 24,000 rpm at 15°C for 48 hours, whereby the precipitate of RNA was obtained. After this precipitate of RNA was dissolved in 0.25% sodium N-lauroylsarcosinate solution, precipitation with ethanol was carried out, whereby 10 mg of RNA was obtained. The RNA thus obtained was adsorbed on an oligo(dT) cellulose column in a high salt solution [0.5 M NaCl, 10 mM Tris-HCl (pH 7.6), 1 mM EDTA and 0.3% SDS], and eluted with a low salt solution [10 mM Tris-HCl (pH 7.6), 1 mM EDTA and 0.3% SDS], to obtain 300 µg of mRNA containing poly(A).

The mRNA thus obtained was further precipitated with ethanol, and dissolved in 0.2 ml of a solution [10 mM Tris-HCl (pH 7.6), 2 mM EDTA and 0.3% SDS]. After treatment at 65°C for 2 minutes, fractionation by sucrose density gradient centrifugation (by a Beckmann SW28 rotor, at 25,000 rpm at 20°C for 21 hours) was conducted. For each of the fractions, a part of RNA was injected into oocyte cells of Xenopus laevis, and the LT activity in protein to be synthesized was measured. As a result, the activity of LT was detected in the fraction corresponding to a sedimentation constant of about 16S. The amount of LT mRNA in this fraction was about 25 µg.

Using the poly(A) RNA as a template, a cDNA library was prepared by using pcDVL vector and pL1 linker according to the method of Okayama and Berg [Mol. Cell. Biol., 2, 161 (1982); ibid., 3, 280 (1983)]. Escherichia coli DH1 was infected with circular vector plasmids containing cDNA, and a cDNA library of about $5 \times 10^5$ clones of which the host was Escherichia coli DH1 was obtained from 5 µg of poly(A) RNA.

(2) Isolation of Plasmid Containing hLT cDNA and Determination of Nucleotide Sequence Thereof

The above human cDNA library using Escherichia coli DH1 was inoculated to 10 nitrocellulose filters (HATF filter manufactured by Millipore) so as to reach $3 \times 10^4$ clones/filter. Twenty (20) replica filters (each pair consisting of 2 filters) were prepared from the above 10 master filters. Plasmid DNA exposed for denaturation by lysing Escherichia coli on the replica filter with 0.5 N NaOH solution was dried for fixation on the filter [M. Grunstein and D. S. Hogness, Proc. Natl. Acad. Sci. U.S.A., 72, 3961 (1975)].

An oligonucleotide having the following formula, which corresponded to a portion (the gene portion corresponding to amino acid No. 162-167) of the nucleotide sequence of the LT gene already reported [Gray et al., Nature, 312, 721 (1984)], was synthesized and used as a screening probe for human LT cDNA.

$^5$ TCCAAAGAAGACAGTACT$^3$

The 5'-terminus of the oligonucleotide probe was labeled with $^{32}$P by using T4 polynucleotide kinase and [$\gamma$-$^{32}$P]ATP.

The labeled probe was hybridized with each of the replica filters on which DNA was fixed. The hybridization reaction was carried out at 40°C for 16 hours in 10 ml of a solution of 5 X SSC (0.15 M NaCl, 0.015 M sodium citrate), 5 X Denhardt's, 0.1% SDS and 100 μg/ml of denatured salmon sperm DNA containing 10 μCi of the labeled probe. After completion of the reaction, the filters were washed with a solution of 6 X SSC and 0.1% SDS at room temperature for 30 minutes 3 times and further at 43°C for 60 minutes twice [the experimental textbook (1), p. 309]. Radioautograms were taken from the washed filters and the radioautograms of the replica filters in sets of two filters were put together in layers for detecting the cells reactive to the probe. By this method, 50 Escherichia coli DH1 strains reactive to the probe were obtained from about $3 \times 10^5$ colonies.

The plasmid DNA was extracted and purified from these cells by the alkaline method [C. H. Birnboim and J. Doly, Nucleic Acids Res., 7, 1513 (1979)]. The DNA was cleaved by the restriction enzyme BamHI (Takara Syuzo) and fractionated by agarose gel electrophoresis. Thereafter, the DNA fragments were transferred on a nitrocellulose filter (BA85 manufactured by S & S) [Southern blotting method, the experimental textbook (1), p. 382]. When this filter was hybridized with the oligonucleotide probe described above, the plasmid DNA fragments reacted with the probe.

Then, one strain of Escherichia coli K12 DH1/pTB618 having the plasmid which had the largest BamHI DNA fragments (cDNA portions) among others was selected. The nucleotide sequence of the cDNA portion of this plasmid DNA was determined by the dideoxynucleotide synthetic chain termination method [J. Messing et al., Nucleic Acids Res., 9, 309 (1981)].

As a result, it was proved that the LT gene contained in plasmid pTB618 was not complete and included from the non-translated portion on the 3'-terminal side upstream to the third C of codon CCC of Pro which was the 18th amino acid.

(3) Construction of hLT (21-171) Expression Vector pTB622

The plasmid pTB618 prepared above was cleaved by restriction enzymes NsiI (Takara Syuzo) and BamHI. and a DNA fragment of 1.1 kilobase pair (hereinafter referred to as Kbp) containing the LT gene was separated. T4 DNA polymerase (PL Inc.) was reacted with the DNA fragment to change the termini of the product to flush ends. Thereafter, a 16-mer EcoRI linker including an ATG translation initiation codon (AACATGAATTCATGTT) was ligated therewith by T4 DNA ligase for adjusting the frame. After T4 DNA ligase was inactivated by heat treatment at 65°C for 10 minutes, digestion was carried out with restriction enzyme EcoRI, and a DNA fragment of 0.6 Kbp containing the LT gene linked with the linker was separated by agarose electrophoresis.

The plasmid ptrp781 described in T. Kurokawa et al., Nucl. Acids Res. 11, 3077 (1983) was cleaved by restriction enzyme EcoRI, and the phosphate of the 5'-terminus was removed by alkaline phosphatase treatment. The thus obtained DNA fragment was mixed with the LT DNA fragment of 0.6 Kbp linked with the EcoRI linker including the ATG codon, and T4 DNA ligase was allowed to act on the mixture, whereby human LT expression vector pTB622 for Escherichia coli was constructed in which the LT gene was inserted downstream of the tryptophan promoter.

(4) Expression of Human LT (21-171) in Escherichia coli Cells, Extraction from Cells and Purification

Escherichia coli strain described in Reference Example 3 was transformed by using plasmid pTB 622 prepared as described above.

The transformant thus obtained was cultivated in 2.5 l of M9-CA medium [the experimental textbook (1), p. 69] at 37°C for 4 hours. After 25 μg/ml of indolylacrylic acid was added thereto, cultivation was further continued for 4 hours. The cells were collected and suspended in 100 ml of Tris-hydrochloric acid buffer (pH 7.5) containing 0.01% lysozyme and 10% sucrose. The suspension was reacted at 5°C for 1 hour, and then treated with ultrasonication for 5 minutes under ice cooling. After centrifugation, the extract was added to a DEAE-Sepharose CL-6B column (Pharmacia) equilibrated with 5 mM phosphate buffer (pH 8.0), and washed with the same buffer, followed by elution with the same buffer containing 0.1 M NaCl to provide a roughly purified solution having a specific activity of $7.8 \times 10^5$ U/mg.

After adjusting to pH 6.0 by hydrochloric acid, the crudely purified solution described above was added to a Blue Sepharose CL-6B column equilibrated with 5 mM phosphate buffer (pH 6.0) containing 0.1 M NaCl, and thoroughly washed, followed by elution with 5 mM phosphate buffer (pH 8.0) containing 0.5 M NaCl. The specific activity of the eluate was $7.4 \times 10^6$ U/mg.

The eluate was further subjected to gel filtration by a Sephacryl S-200 column equilibrated with 5 mM phosphate buffer (pH 7.3) to obtain a purified solution having a specific activity of $1.6 \times 10^7$ U/mg.

Reference Example 5

ELISA for Determining Anti-hLT Antibody

The 5μg/ml hLT solution obtained in Reference Example 4-(4) was added to a microplate having 96 wells in an amount of 100μl/well and allowed to stand at 4°C for a day and night. Further, 20 mM phosphate buffered saline (PBS, pH 7.3) containing 2% bovine serum albumin (BSA) was added thereto to prepare a sensitized plate. When the ELISA was carried out, the above solution was removed from the microplate and the microplate was washed with PBS. Then, a test hybridoma culture supernatant was added thereto and reacted at room temperature for 2 hours. After washing with PBS, HRP-labeled rabbit anti-mouse IgG antibody was added thereto and further reacted at room temperature for 2 hours.

After washing, there was added to each well 0.1 M citrate buffer containing o-phenylenediamine and $H_2O_2$, and the enzyme reaction was conducted at room temperature. The reaction was terminated with 1 N sulfuric acid, and then the amount of color-developed dye was determined at 492 nm with Multiscan (Flow Laboratory).

Reference Example 6

ELISA for Determining Anti-HRP Antibody

The 5μg/ml rabbit anti-mouse IgG antibody solution was added to a microplate having 96 wells in an amount of 100 μl/well and allowed to stand at 4°C for a day and night. Further, 20 mM PBS containing 2% BSA was added thereto to prepare a sensitized plate. When ELISA was carried out, the above solution was removed from the microplate and the microplate was washed with PBS. Then, a test hybridoma culture supernatant was added thereto and reacted at room temperature for 2 hours. After washing with PBS, HRP was added thereto and further reacted at room temperature for 2 hours. Thereafter, according to the method described in Reference Example 5, enzyme reaction was conducted and the antibody titer was determined.

Reference Example 7

ELISA for Determining Hybrid Antibody

A test hybridoma culture supernatant was added to the hLT-sensitized plate prepared in Reference Example 5 and reacted at room temperature for 2 hours. After washing with PBS, HRP was added thereto and further reacted at room temperature for 2 hours. Thereafter, according to the method described in Reference Example 5, the enzyme reaction was conducted and the antibody titer was determined.

11

Reference Example 8

Preparation of Mouse Anti-Human LT Monoclonal Antibody

(1) Immunization

To 200 μg of the purified human LT sample described in Reference Example 4-(4) in 1 ml of a physiological saline, the same volume of Freund's complete adjuvant was added and fully emulsified. The emulsion was then administered to BALB/c mice ( ♀, n = 10 : 20 μg/0.2 ml/mouse) intraperitoneally and subcutaneously in their backs, and booster immunization was carried out at intervals of 3 weeks. After the booster immunization was conducted 4 times, the same LT antigen solution (50 μg/0.1 ml of physiological saline mouse) was intravenously administered to the individual which showed the highest serum antibody titer after 2 weeks.

(2) Cell Fusion

The spleen was taken out of the mouse 3 days after the final immunization, and a spleen cell suspension containing about $10^8$ cells was prepared by a conventional method. Then, 2 X $10^7$ mouse myeloma cells (P3UI) were added thereto, and cell fusion was conducted by using PEG 6000 according to the method of Köhler and Milstein [Nature, 256, 495 (1975)].

After completion of the fusion, the cell mixture was suspended in HAT medium containing hypoxanthine, aminopterin and thymidine, and cultivated for 10 days. Then, immediately after selection of parent cells was completed, HAT medium was substituted for HT medium from which aminopterin was eliminated, and the cultivation was continued.

(3) Selection and Cloning of Hybridoma

The antibody titer of hybridoma culture supernatants was determined by the ELISA using a microplate in which purified human LT was adsorbed on a solid phase. After 10 to 20 days from the fusion, hybridomas and an antibody which could bind to human LT were observed. The hybridomas having particularly high binding activity were cloned by the limiting dilution method.

Similarly, the cloned hybridoma culture supernatants were screened by the ELISA, and the supernatants having high binding activity to human LT were selected. For these supernatants, the neutralization ability against the human LT activity was measured by using the test of the cytotoxic activity against L929 cells described in Reference Example 1. Namely, to 100 units/ml of human LT, the same amount of the hybridoma culture supernatant was added and reacted for 1 hour, followed by the test of the cytotoxic activity against L929.

As a result, there were obtained MoAB-producing hybridomas LT3-11 and LT3-135 which were capable of linking with human LT and neutralizing the cytotoxic activity against them. Their immunoglobulin class and subclass were examined by the Ouchterlony test. They were found to belong to $IgG_{2b}$ and $IgG_{2a}$, respectively.

Reference Example 9

Preparation of Rabbit Anti-Human LT C-Terminal Peptide Antibody

(1) Preparation of Immunogen

A 10 mg/5 ml aqueous solution of the human LT C-terminal peptide (152-171) prepared by using a peptide synthesizer (Model 430A manufactured by Applied Biosystems) according to a known solid phase synthesis method was added to a 40 mg/5 ml aqueous solution of bovine thyroglobulin (BTG). After slight ultrasonication, 1 ml of a 2% glutalaldehyde(GLA) solution was gently added dropwise thereto under ice cooling and reacted for 5 hours. The mixture was dialyzed 3 times against every 3 l of a physiological saline, and stored under a frozen condition for use as an immunogen.

(2) Immunization

To 4 mg of the peptide-BTG conjugate in 1.5 ml of a physiological saline, the same volume of Freund's complete adjuvant was added. The mixture was subcutaneously administered to rabbits (♂, n = 3: 1.3 mg/1 ml/rabbit) at their backs and hind-limb palms to initiate immunization. Booster immunizations were carried out by inoculating the immunogen to which the same volume of Freund's incomplete adjuvant was added, 5 times at 4-week intervals.

(3) Preparation of Antibodies

After 7 to 10 days from the final immunization, the blood is collected from the ear veins to obtain the antiserum by centrifugation.

Specific antibodies were prepared by subjecting the above-mentioned serum to salting out and column chromatography and further purifying the obtained antibody IgG fraction by affinity chromatography using an insolubilized human LT-cellulofine column, according to a known method. That is to say, the rabbit anti-human LT-IgG fraction was added to a human LT-linked column equilibrated with 0.02 M borate buffer (pH 8.0) containing 0.15 M NaCl, and thoroughly washed, followed by elution with 0.02 M glycine-hydrochloric acid buffer (pH 2.3). Thus, specific neutralizing antibodies LT-R1, LT-R2 and LT-R3 having high affinity for human LT were obtained.

Example 1

Preparation of Bispecific Hybrid Monoclonal Antibody

(1) Immunization

To a solution containing 200 μg of commercial HRP (sold by Seikagaku Kogyo) per ml of physiological saline, the same volume of Freund's adjuvant was added and fully emulsified. The emulsion was then administered to BALB/c mice (♀, 20 μg/0.2 ml/mouse) intraperitoneally and subcutaneously at their backs, and booster immunizations were carried out at an interval of 2 to 3 weeks. After the booster immunizations were conducted 3 times, an HRP antigen solution (50 μg/0.1 ml of physiological saline/mouse) was intravenously administered to the individual which showed the maximum serum antibody titer after 10 days.

(2) Cell Fusion

The hLT-neutralizing antibody-producing hybridoma LT3-11 obtained in Reference Example 8 was cultivated and conditioned with 8-AZG-added medium to turn it HAT- sensitive. Namely, cultivation was initiated with Iskove-Ham F12 mixture medium containing 1 μM 8-AZG and 10% FCS, and continued while increasing concentrations of 8-AZG two-to five-fold at a time successively from 1 μM. For strains which became sensitive to 100 μM of 8-AZG, the HAT sensitivity was examined, and thereby anti-hLT antibody-productive and HAT-sensitive strain LTAG-1 was obtained.

The spleen cells of the HRP-immunized mice obtained in (1) were added to the above LTAG-1 in a ratio of 1 : 5 and fused with each other in such a manner as described in Reference Example 8-(2). The obtained trioma cells were selectively cultivated with HAT medium. As a result, the growth of the trioma cells was observed after 10 to 15 days from the cell fusion.

(3) Selection and Cloning of Trioma

13

The antibody titer of the culture supernatants was determined by the ELISAs described in Reference Examples 5, 6 and 7. Cloning was performed for 3 wells which was positive in all of the assay systems. As a result of the cloning by the limiting dilution method, trioma (hybrid hybridoma) PLT-109 strain was obtained which showed the maximum hybrid antibody activity in the ELISA described in Reference Example 7.

(4) Purification of Hybrid Antibody

Into 6 BALB/c mice to which 0.5 ml of mineral oil had been intraperitoneally administered, $5 \times 10^6$ cells/mouse of trioma PLT1-109 was intraperitoneally injected. After 10 to 20 days, about 20 ml of pooled ascites was collected and salted out with 45-50% saturated ammonium sulfate to obtain an IgG fraction.

Then, the fraction was equilibrated with 20 mM PBS (pH 8.0) and subjected to a Protein A column to obtain a protein fraction which was eluted with 0.1 M acetate buffer, followed by application to affinity chromatography using a HRP-combined cellulofine column. An anti-HRP-specific antibody fraction which was eluted with 0.05 M glycine-hydrochloric acid buffer (pH 2.3) was collected, and then applied to high performance liquid chromatography using a hydroxyapatite column to isolate 4.2 mg of bispecific hybrid antibody PLT1-109 of the present invention.

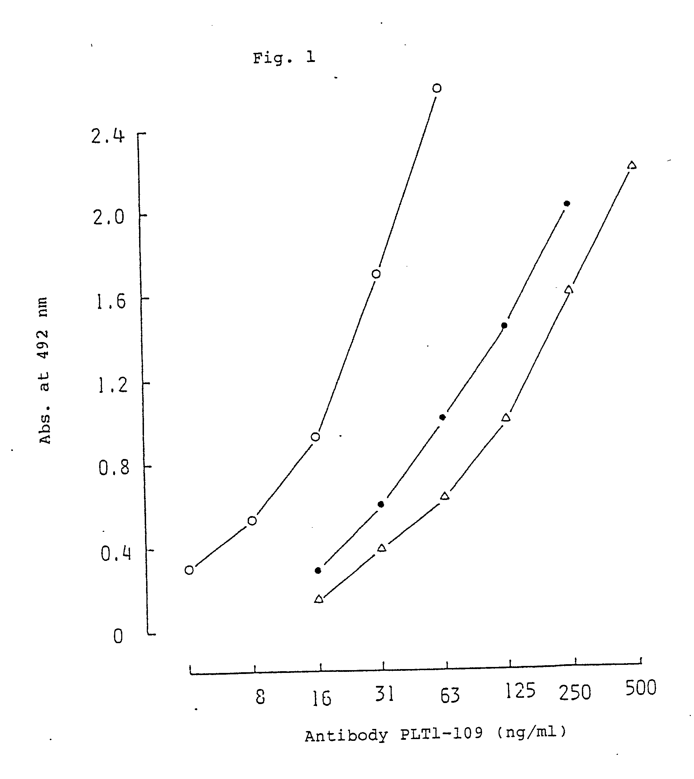

The hybrid antibody described above was subjected to the ELISAs described in Reference Examples 5 to 7. The results shown in Fig. 1 were obtained.

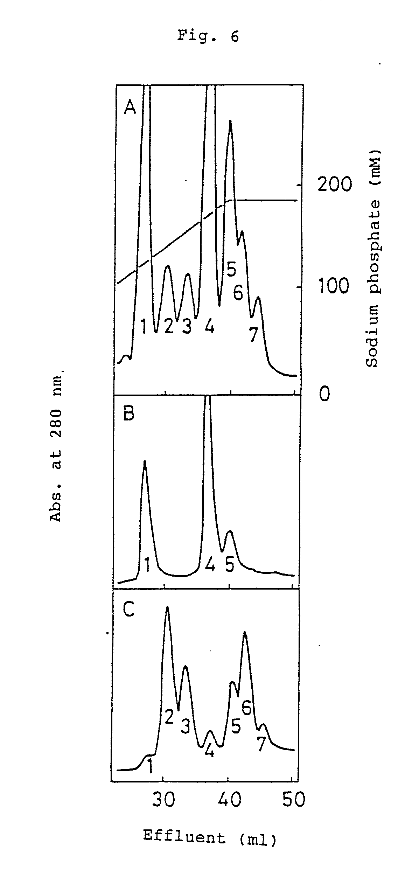

High performance liquid chromatograms obtained by use of the above hydroxyapatite column are shown in Figs. 6(A), 6(B) and 6(C). Figs. 6(A), 6(B) and 6(C) show the high performance liquid chromatograms of the IgG fraction obtained by salting out with ammonium sulfate, the fraction eluted at pH 2.3 on the HRP-combined column and a fraction passing at pH 8.0 on the HRP-combined column, respectively.

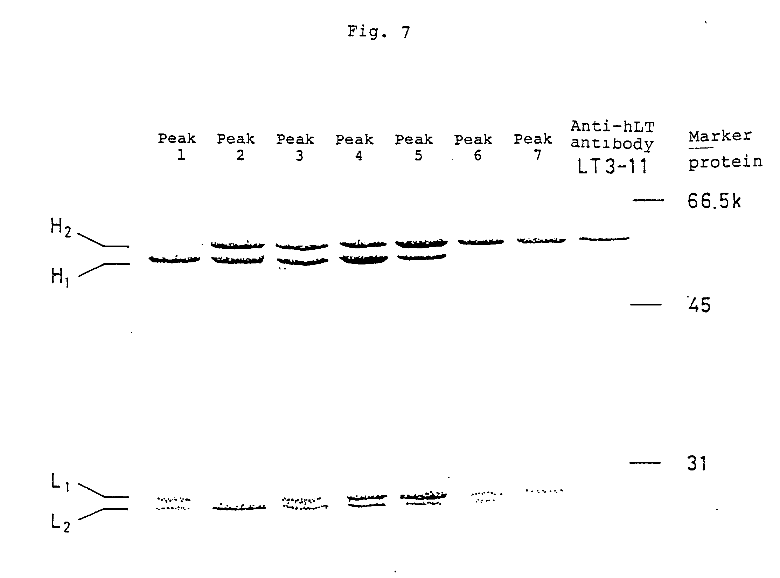

Fig. 7 shows the results obtained in subjecting the peaks 1 to 7 [see Fig. 6(A)] and parent anti-hLT MoAb LT3-11 to sodium dodecyl sulfate-polyacrylamide electrophoresis (SDS-PAGE) according to the method of Laemmli [U. K. Laemmli, Nature, 227, 680 (1974)]. If the anti-HRP antibody is represented by H·L·-H·L· and the anti-hLT MoAb LT3-11 is represented by $H_2L_2$-$H_2L_2$, it can be seen that hybrid antibodies are composed of two kinds of H chains ($H_1$ and $H_2$) and L chains ($L_1$ and $L_2$)(see Table 1).

Table 1 shows the results obtained in subjecting the peaks 1 to 7 [see Fig. 6(A)] to the ELISAs described in Reference Examples 5 to 7 and immunoglobulin species included in the peaks. Each peak was identified on the basis of the chromatograms shown in Fig. 6 and the results of the SDS-PAGE shown in Fig. 7. The bispecific hybrid MoAB of the present invention is included in the peak 4.

Table 1

| Peak | Peak area(%) | ELISA antibody titer | | | Included Ig species |
|------|-------------|---------------------|---------------------|----------------------|---------------------|
| | | Anti-HRP antibody | Anti-hLT antibody | Bispecific antibody | |
| Peak 1 | 22 | 512 | <100 | <100 | $H·L_2$-$H·L_2$ $H·L_1$-$H·L_2$ $H·L_1$-$H·L_1$ |
| Peak 2 | 8 | <100 | 256 | <100 | $H·L_2$-$H_2L_2$ |
| Peak 3 | 7 | <100 | <100 | <100 | $H·L_2$-$H_2L_1$ |
| Peak 4 | 34 | 1024 | 1024 | 256 | $H·L_1$-$H_2L_2$ |
| Peak 5 | 16 | 256 | 256 | <100 | $H_1L_1$-$H_2L_1$ $H_2L_2$-$H_2L_2$ |
| Peak 6 | 8 | <100 | 256 | <100 | $H_2L_1$-$H_2L_2$ |
| Peak 7 | 5 | <100 | 100 | <100 | $H_2L_1$-$H_2L_1$ |

Example 2

hLT ELISA (1) Using Hybrid Antibody

To each well of the hLT-sensitized plate prepared in Reference Example 5, 50 μl portions of an hLT-containing test solution diluted with PBS containing 2% BSA, a hybrid antibody solution (PLT1-109, 125 ng/ml) and an HRP solution (25 μg/ml) were added, and the reaction was conducted at room temperature for 1 hour. The plate was thereafter washed with PBS containing 0.1% Tween 20. Then, the enzyme reaction was conducted according to the method described in Reference Example 5, followed by determination of the concentration of hLT.

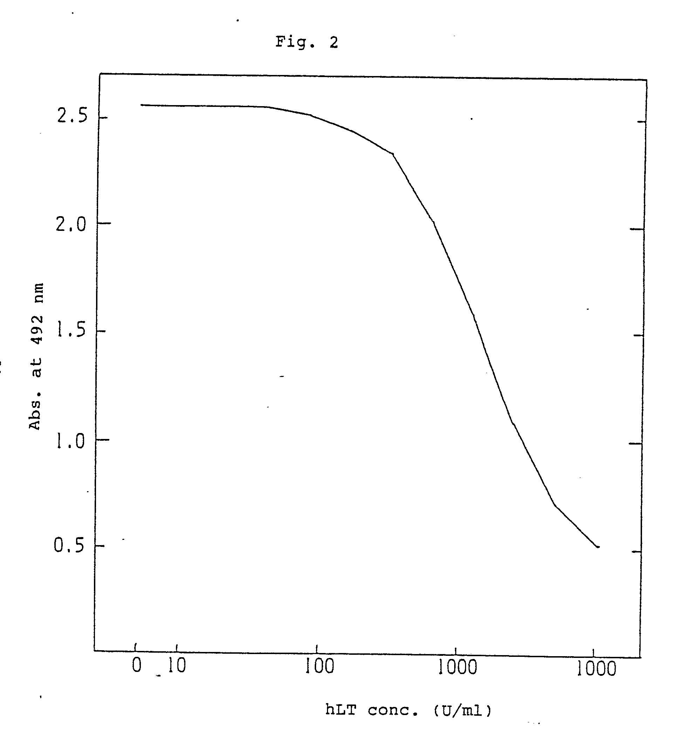

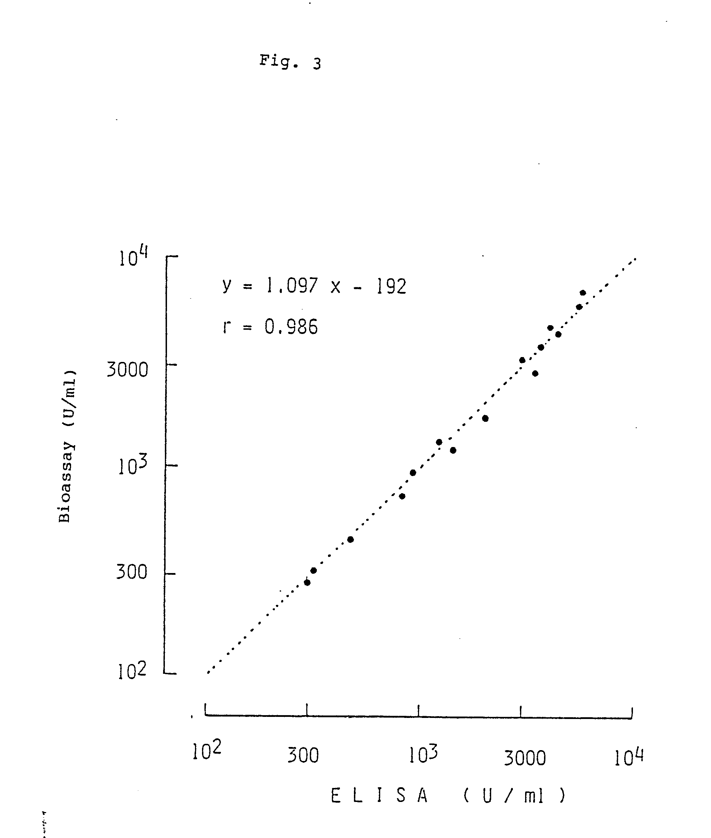

Fig. 2 shows a standard curve of hLT. Fig. 3 shows the results obtained in comparing the ELISA of the present invention with the bioassay of Reference Example 1 in which the lymphocyte culture supernatant obtained in Reference Example 2 and the Escherichia coli cell-extract obtained in Reference Example 4-(4) were added. The correlation coefficient (r) obtained with respect to 15 specimens was 0.986, which was satisfactory.

Example 3

hLT ELISA (2) Using Hybrid Antibody

The 25 μg/ml solution of the purified hLT C-terminal peptide-specific antibody prepared in Reference Example was added to a microplate having 96 wells in an amount of 100 μl/well and allowed to stand at 4°C for a day and night. Further, PBS containing 2% BSA was added thereto to prepare a sensitized plate. The plate was washed with PBS, and 100 μl of an hLT-containing test solution was added thereto, followed by reaction at room temperature for 1 hour. Then, the plate was washed again with PBS containing 0.1% Tween 20. Subsequently, 50 μl portions of a hybrid antibody solution (PLT1-109, 250 ng/ml) and an HRP solution (25 μg/ml) were added thereto, and the reaction was conducted at room temperature for 1 hour. The plate was thereafter washed with PBS containing 0.1% Tween 20. Then, the enzyme reaction was conducted according to the method described in Reference Example 5, followed by preparation of a standard curve of hLT.

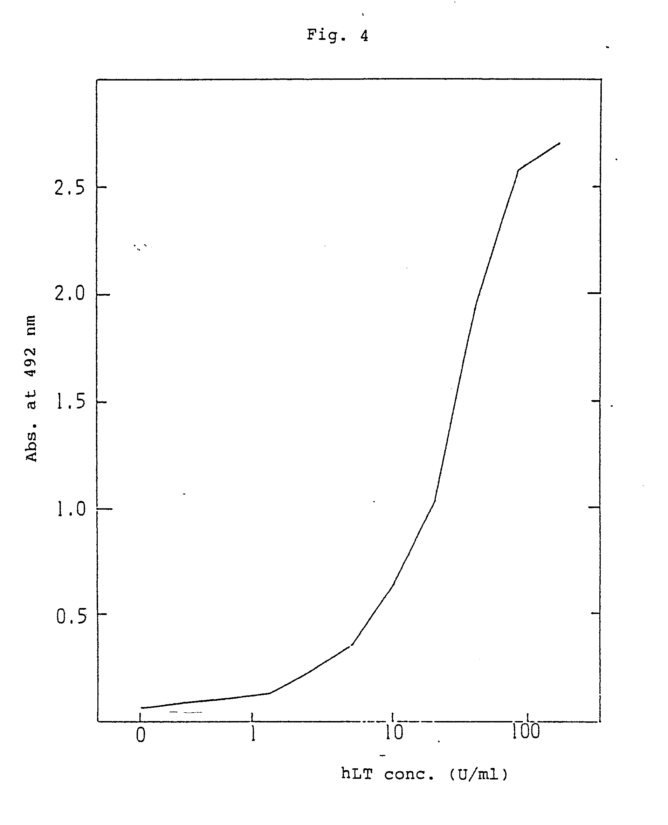

The results are as shown in Fig. 4.

Example 4

hLT-ELISA (3) Using Hybrid Antibody

To the hLT-C-terminal peptide antibody-sensitized plate described in Example 3, 50 μl of an hLT-containing test solution, 25 μl of a hybrid antibody solution (PLT1-109, 500 ng/ml) and 25 μl of an HRP solution (50 μg/ml) were added, and the reaction was conducted at room temperature for 1 hour. The plate was thereafter washed with PBS containing 0.1% Tween 20. Then, the enzyme reaction was conducted according to the method described in Reference Example 5, followed by preparation of a standard curve of hLT.

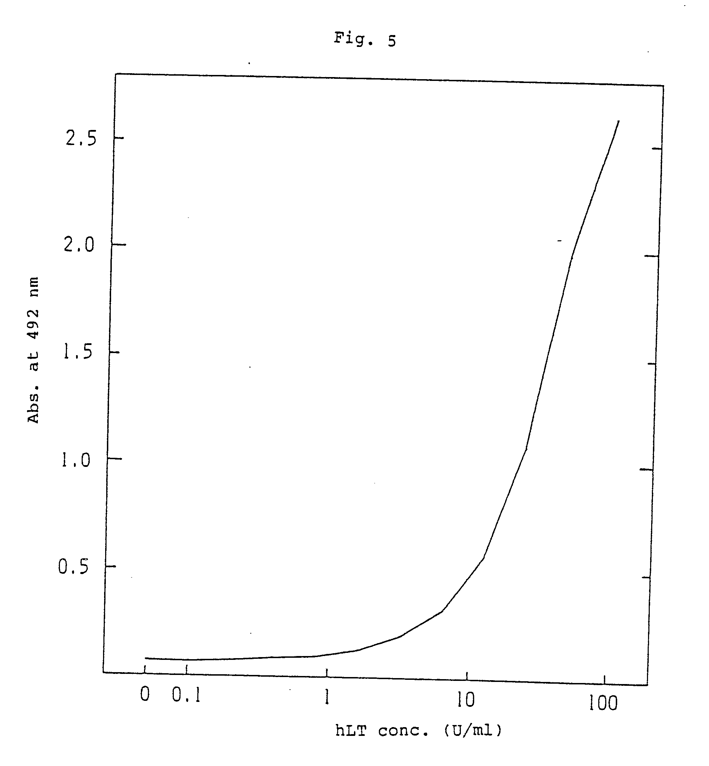

The results are as shown in Fig. 5.

Example 5

hLT-ELISA (4) Using Hybrid Antibody

To the plate sensitized with the hLT C-terminal peptide antibody described in Example 3, 50 μl of an hLT-containing test solution and 50 μl of a hybrid antibody (25 ng) solution previously mixed with HRP (1.25 μg) were added, and the reaction was conducted at room temperature for 1 hour. The plate was thereafter washed with PBS containing 0.1% Tween 20. Then, the enzyme reaction was conducted according to the method described in Reference Example 5, followed by determination of the concentration of hLT in the lymphocyte culture supernatant obtained in Reference Example 2 and the Escherichia coli cell-extract obtained in Reference Example 4-(4).

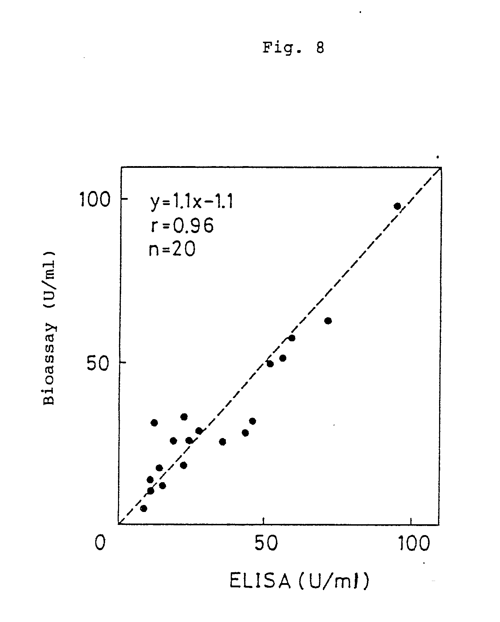

Fig. 8 shows the results obtained in comparing the ELISA of the present invention with the bioassay of Reference Example 1. The correlation coefficient (r) obtained with respect to 20 specimens was 0.96, which was satisfactory.

Example 6

hLT-Neutralizing Activity of Hybrid Antibody

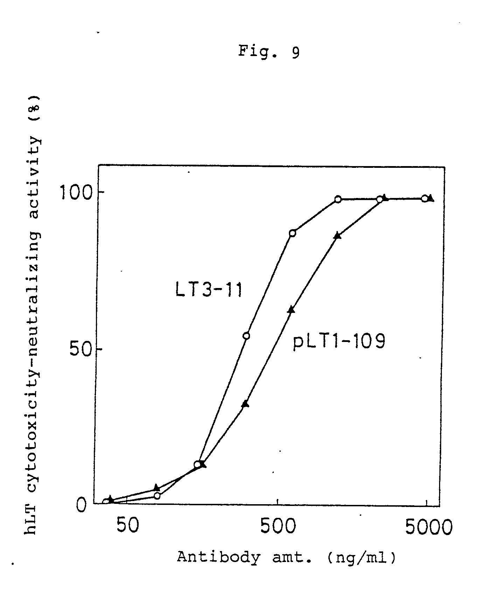

The hLT-neutralizing activity of anti-hLT MoAb LT3-11 and bispecific hybrid MoAb PLT1-109 was determined, using the test for neutralizing the cytotoxic activity against L929 described in Reference Example 8-(3). The results are as shown in Fig. 9. The hLT-neutralizing activity of hybrid MoAb PLT1-109 of the present invention was 50 to 70% of that of the parent antibody, LT3-11, and the neutralizing activity per valence of the former was nearly equal to that of the latter.

Claims

1. A polydoma which produces a bispecific hybrid monoclonal antibody specific for both human lymphotoxin and a substance which permits detection of the human lymphotoxin.

2. The polydoma according to claim 1, wherein the substance which permits detection of the human lymphotoxin is an enzyme, a fluorescent substance or a radionuclide.

3. The polydoma according to claim 2, wherein the enzyme is horseradish peroxidase.

4. A trioma which produces a bispecific hybrid monoclonal antibody which binds both to human lymphotoxin and to a substance which permits detection of the human lymphotoxin, prepared by fusion of an anti-human lymphotoxin antibody-producing hybridoma with an antibody-producing cell obtained from a mammal immunized with said substance.

5. The trioma according to claim 4, wherein the substance which permits detection of the human lymphotoxin is an enzyme, a fluoroescent substance, or a radionuclide.

6. The trioma according to claim 4, wherein the substance which permits detection of the human lymphotoxin is horseradish peroxidase.

7. The trioma according to claim 4, wherein the mammal is a mouse.

8. The trioma as recited in claim 4, wherein the anti-human lymphotoxin antibody-producing hybridoma is LT3-11 or LT3- 135.

9. A trioma prepared by fusion of the hybridoma LT3-11 with an antibody-producing cell obtained from a mouse immunized with horseradish peroxidase.

10. The trioma PLT1-109.

11. A hybrid monoclonal antibody produced from the polydoma of claim 1, which comprises one molecule capable of linking both to human lymphotoxin and to horseradish peroxidase, which permits detection of human lymphotoxin.

12. A hybrid monoclonal antibody produced from the trioma of claim 10, which comprises one molecule capable of linking both to human lymphotoxin and to horseradish peroxidase, which permits detection of human lymphotoxin.

13. An immunodiagnostic method for detection of human lymphotoxin in a test solution, which comprises subjecting (1) said test solution and (2) human lymphotoxin which is held on a carrier to a reaction with (3) a hybrid monoclonal antibody capable of linking both to human lymphotoxin and to a substance which permits detection of the human lymphotoxin, to react human lymphotoxin in said test solution and human lymphotoxin on said carrier competitively with said hybrid monoclonal antibody.

14. An immunodiagnostic method for detection of human lymphotoxin in a test solution, which comprises subjecting (1) a test solution to a reaction with (2) a hybrid monoclonal antibody capable of linking both to human lymphotoxin and to a substance which permits detection of the human lymphotoxin and (3) an anti-human lymphotoxin antibody which has a human lymphotoxin recognition site different from that of said hybrid monoclonal antibody and which is held on a carrier, to react human lymphotoxin in said test solution with said anti-human lymphotoxin antibody and said hybrid monoclonal antibody.

Fig. 1

Fig. 2

Fig. 3

$$y = 1.097 \; x \; - \; 192$$

$$r = 0.986$$

Fig. 4

hLT conc. (U/ml)

Fig. 5

EP 0 338 497 A2

Fig. 6

Fig. 7

Fig. 8

Fig. 9